# EUROPÄISCHE PATENTANMELDUNG

(11) **EP 0 638 618 A2**
(43) Veröffentlichungstag der Anmeldung: **15.02.1995**
(21) Anmeldenummer: 94112335.8
(22) Anmeldetag: 08.08.1994
(51) Int. Cl.: C09D 11/00

(54) **Magnetisches Tintenkonzentrat**

(30) Priorität: 13.08.1993 DE 4327225
(71) Anmelder: BASF Aktiengesellschaft, D-67063 Ludwigshafen (DE)
(72) Erfinder: Kormann, Claudius, Dr., D-67105 Schifferstadt (DE); Schwab, Ekkehard, Dr., D-67434 Neustadt (DE); Sinn, Ekkehard, Dr., D-07743 Jena (DE); Müller, Robert, D-07749 Jena (DE); Goernert, Peter, Prof. Dr., D-07747 Jena (DE)

(57) **Zusammenfassung**

Beschrieben ist ein magnetisches Tintenkonzentrat, im wesentlichen bestehend aus einer Dispersion hartmagnetischer Feststoffteilchen in Wasser oder Alkohol in Gegenwart von Polyelektrolyten mit einem Molekulargewicht zwischen 1000 und 25000, ausgewählt aus der Gruppe von Polyacrylaten, Acrylsäure-/Acrylamid-Copolymeren und Polyvinylphosphonsäuren sowie der Alkalisalze dieser Verbindungen mit der Maßgabe, daß die magnetischen Pigmente eine spezifische Oberfläche von 70 bis 200 m²/g, eine Koerzitivkraft zwischen 10 und 500 kA/m sowie eine remanente Magnetisierung von über 10 nTm³/g aufweisen. Als Polyelektrolyte sind auch biologisch abbaubare Polykondensate aus organischen Hydroxycarbonsäuren, Alkoholen oder Acetalen anwendbar. Das magnetische Tintenkonzentrat läßt sich sehr einfach in einem Arbeitsgang herstellen.

## Beschreibung

Die Erfindung betrifft ein magnetisches Tintenkonzentrat, im wesentlichen bestehend aus einer Dispersion magnetischer Feststoffteilchen in Wasser oder Alkohol in Gegenwart dispergierender Substanzen, wobei diese aus mindestens einem Polyelektrolyten mit einem Molekulargewicht zwischen 1000 und 25000 ausgewählt aus der Gruppe aus Polyacrylat, Acrylsäure-/Acrylamid-Copolymeren und Polyvinylphosphonsäure sowie der Alkalisalze dieser Verbindung besteht.

Ein magnetisches Tintenkonzentrat der oben genannten gattungsmäßigen Art ist bereits aus der DE-OS 41 15 608 bekannt. Dabei werden superparamagnetische Feststoffteilchen in Wasser oder Alkohol und dispergierend wirkenden Polyelektrolyten mit einem Molekulargewicht zwischen 1000 und 25000 beschrieben. Diese Tintenkonzentrate eignen sich für den Einsatz in Tintenstrahldruckern und ähnlichen Schreibgeräten. Die damit erzeugten magnetischen Druckmerkmale sind weichmagnetisch, das heißt, sie können im Magnetfeld aufmagnetisiert und über eine Suszeptibilitätsmessung detektiert werden. Jedoch verlieren die Druckmerkmale nach Ausschalten des Magnetfeldes ihre magnetische Eigenschaft; für eine große Zahl von Anwendungen ist es jedoch zur Informationsspeicherung erforderlich, mit einem Tintenstrahldrucker magnetische Schriftzeichen mit Remanenz zu erzeugen, beispielsweise Ziffern auf Schecks, Banknoten sowie Magnetstreifen auf U-Bahn-Tickets und so weiter. Die bekannten magnetischen Suspensionen, welche hartmagnetische Pigmente in einem polymeren Bindemittel dispergiert enthalten, wie sie zur Beschichtung von konventionellen magnetischen Aufzeichnungsträgern hergestellt werden, eignen sich jedoch nicht für die Anwendung im Tintenstrahldrucker oder ähnlichen auf der Wirkung kapillarer Kräfte beruhenden Geräten, da die Viskosität und die Sedimentationsneigung der Suspension zu groß sind. Diese Eigenschaft rührt von der Bildung von Agglomeraten in den Dispersionen her. Aufgrund ihrer Remanenz verhalten sich die Pigmente wie kleine Dauermagnete, die sich anziehen, zusammenballen und als Agglomerat wegen der hohen Dichte sedimentieren. Auch enthalten die bekannten Magnetsuspensionen in der Regel leicht flüchtige hochbrennbare Lösungsmittelbestandteile in hoher Konzentration, welche besondere Maßnahmen bezüglich Explosionsgefahr und Umweltschutz erfordern.

Es bestand also die Aufgabe, ein magnetisches Tintenkonzentrat der oben genannten gattungsmäßigen Art zu finden, welches zu Markierungen mit Remanenz führt sowie einfach aufgebaut und herzustellen ist.

Es wurde überraschend gefunden, daß diese Aufgabe sich dadurch lösen läßt, daß ein magnetisches Tintenkonzentrat mit den im kennzeichnenden Teil des Anspruchs 1 oder 2 genannten Merkmalen eingesetzt wird. Weitere Einzelheiten der Erfindung gehen aus den Unteransprüchen sowie der Beschreibung und den Beispielen hervor.

Das Erfindungswesentliche besteht darin, daß man in einem einstufigen Prozeß einen mit Wasser oder Alkohol gefeuchteten Filterkuchen eines feinteiligen hartmagnetischen Pigments mit einer Lösung eines Polyelektrolyten vermischt und dispergiert, wobei die Pigmente und die Polyelektrolyten die weiter unten im folgenden näher dargestellten Randbedingungen erfüllen müssen.

An sich ist die Verwendung von feinteiligen hartmagnetischen Pigmenten für Ferrofluide beziehungsweise Tintenkonzentrate aus dem Stand der Technik bekannt, beispielsweise aus der DE 30 27 012, in der die Zusammensetzung und Herstellung von Ferrofluiden, welche in Wasser dispergiert sind, beschrieben werden. Weiterhin ist aus dem Artikel "Synthesis and magnetic properties of manganese and cobalt ferrite ferrofluids" (Progress in Colloid & Polymer Science, 1989, Band 79, Seite 128-134) die Herstellung neuerer Ferrofluide beschrieben. Diese weist jedoch einen relativ komplizierten insgesamt vierstufigen Prozeß auf, der unter anderem Wässerungsschritte beinhaltet und damit zu einer erheblichen Abwasserbelastung führt. Die so hergestellten Ferrofluide haben einfach geladene Gegenionen, während das magnetische Tintenkonzentrat gemäß der vorliegenden Erfindung mindestens fünf Ladungen in einem Polyelektrolyten aufweist.

Schließlich sind die Ferrofluide gemäß dem Stand der Technik meist mit einem entgegengesetzt geladenen Tensid verknüpft, was den Nachteil von Schaumbildung mit sich führt und wobei das Aufsprühen derartiger Tintenkonzentrate nicht möglich ist, da die Tröpfchen zerplatzen, während die Tintenkonzentrate mit den verwendeten Polyelektrolyten keine Schaumtendenz zeigen und demgemäß nicht die oben aufgeführten Nach teile aufweisen.

Die im Rahmen der Erfindung einsetzbaren hartmagnetischen Pigmente können aus der Gruppe der hexagonalen Ferrite beispielsweise mit der Zusammensetzung BaFe_{12-x-y}Mₓ^{II}M_{y}^{IV}O₁₉ oder SrFe_{12-x-y}Mₓ^{II}M_{y}^{IV}O₁₉ sein, wobei x = 0-1, y = 0-1 und M^{II} = Mn, Fe, Co, Ni, Zn, Cu, Mg, Cd und M^{IV} = Ti, Zr, Sn, Nb sein kann. Die Herstellung solcher Pigmente, beispielsweise nach dem Glasverfahren, ist beschrieben in der EP 0 136 599.

Weitere Pigmentherstellverfahren sind in der EP 0 346 123 sowie der JP 04-284 603 vorbeschrieben.

Des weiteren können Pigmente der Zusammensetzung
MₐMn_{b}Zn_{c}Fe_{d}Oₑ verwendet werden, wobei M = Co und/oder Ni und
a, b, c = 0,00 - 0,99 a+b+c = 0,01 - 0,99
d = 2,00 - 2,99 a+b+c+d = 2,00 - 3,00
e = 3,01 - 4
wobei durch geeignete Auswahl der Dotierungselemente eine Koerzitivkraft der Pigmente von mindestens 10 kA/m einzustellen ist. Die Auswahlregeln sind dem Fachmann bekannt. Beispielsweise bewirkt eine Erhöhung des Co-Gehalts eine Erhöhung der Koerzitivkraft. Bevorzugte Co-Gehalte sind im Falle isometrischer Partikel: a = 0,1 - 0,5. Falls jedoch durch bekannte Herstellverfahren erzeugte anisometrische, z. B. nadelförmige Partikel verwendet werden, werden deutlich weniger Dotierstoffe benötigt.

Das bei 80 °C im Vakuum getrocknete Pigment oder die Mischung verschiedener Pigmente soll, im Rahmen der Erfindung eingesetzt, eine spezifische Oberfläche von 70 bis 200 m²/g, vorzugsweise von 90 - 120 m²/g aufweisen. Die Koerzitivkraft des Pigments oder der Mischung verschiedener Pigmente soll zwischen 10 und 500 kA/m, vorzugsweise zwischen 15 und 350 kA/m liegen. Die Remanenz des Pigments oder der Mischung verschiedener Pigmente soll über 10 nTm³/g betragen.

Die spezifische Oberfläche wurde hierbei gemäß DIN 66132 mittels eines Ströhlein-Areameters der Firma Ströhlein, Düsseldorf, nach dem Einpunkt-Differenz-Verfahren nach Haul und Dümbgen bestimmt. Die magnetischen Eigenschaften der Pigmente wurden mit einem Schwingmagnetometer ermittelt. Die Messung erfolgt bei einer maximalen Feldstärke von 380 kA/m, wobei die Koerzitivfeldstärke Hc in kA/m, umgerechnet auf eine Stopfdichte der Meßprobe von 1,2 g/cm³ angegeben ist.

Dem hartmagnetischen Pigment beziehungsweise der Pigmentmischung können auch superparamagnetische Pigmente, wie sie beispielsweise in der bereits genannten DE-OS 41 15 608 beschrieben sind, in einem Mengenanteil bis zu 50 Gew.% zugemischt werden. Ebenso können auch unmagnetische feinteilige Pigmente bis zu einem Gewichtsanteil von bis zu 50 % zugemischt werden, ferner auch Mischungen von superparamagnetischen und unmagnetischen feinen Teilchen. In jedem Fall müssen die Eigenschaften der Mischung die oben angegebenen Randbedingungen bezüglich Koerzitivkraft und Remanenz erfüllen.

Als Trägermedium für die erfindungsgemäßen Tintenkonzentrate werden polare Flüssigkeiten, vorzugsweise die an sich bekannten wie Wasser oder Alkohole mit einer Dielektrizitätskonstanten von mindestens 20, eingesetzt. Als Alkohole seien beispielhaft Ethylenglykol, Diethylenglykol oder Glyzerin genannt, wobei auch Gemische dieser Alkohole mit Wasser eingeschlossen sind.

Die in den erfindungsgemäßen Tintenkonzentraten eingesetzten Polyelektrolyte können die gleichen sein, wie die in der mehrfach genannten DE-OS 41 15 608 beschriebenen, welche ein Molekulargewicht zwischen 1000 und 25000 haben, insbesondere zwischen 1500 und 20000 und vorzugsweise um 4000. Bevorzugt sind Polyacrylsäure, Acrylsäure-/Acrylamid-Copolymere sowie Polyvinylphosphonsäure und/oder deren Alkalisalze. Ebenso erfindungsgemäß einsetzbar sind Polykondensate aus organischen Hydroxy-Carbonsäuren, Hydroxy-Carbonsäuren und Alkoholen oder Acetalen, wobei das Molekulargewicht der Monomere zwischen 100 und 400 liegt und wobei der K-Wert der 1%igen Lösung des Polykondensats in Wasser zwischen 10 und 40 beträgt und wobei im Mittel wenigstens 5 Ladungsträger in der Polymerkette vorliegen. Diese genannten Polykondensate haben den Vorzug, biologisch abbaubar zu sein, ihr Aufbau und ihre Herstellung sind näher in der parallelen deutschen Anmeldung P 43 27 223 der gleichen Anmelderin beschrieben.

Die zugesetzte Menge dieser Substanzen wird nach der spezifischen Oberfläche der hartmagnetischen Pigmente sowie deren Beimischungen bemessen und beträgt mindestens 0,5 mg/m² BET-Oberfläche, wobei sich Mengen zwischen 1,5 und 5 mg/m² als besonders vorteilhaft herausgestellt haben.

Außer diesen Komponenten können die erfindungsgemäßen magnetischen Tintenkonzentrage noch Additive zur Regulierung des Fließverhaltens, wie zum Beispiel Alkylphenolate, enthalten. Auch die Zugabe von Hochsiedern wie Diethylenglykol, Ethylenglykol, Glyzerin und Polyethylenglykol in untergeordneten Mengen läßt sich gegebenenfalls zur Anpassung günstiger Fließ- und Trocknungseigenschaften heranziehen. Durch den Zusatz von Farbstoffen läßt sich, sofern eine gewisse Verringerung der Sättigungsmagnetisierung nicht störend ist, zudem die Farbtiefe der Tintenkonzentrate variieren.

Die erfindungsgemäßen Tintenkonzentrate lassen sich in einfacher Weise herstellen. Der feuchte Filterkuchen mit einem Ferritgehalt von 20 bis 40 Gew.% wird, wie in den nachfolgenden Beispielen näher beschrieben, mit einer wässrigen oder alkoholischen Lösung des Polyelektrolyten mit einem Feststoffgehalt von 25 bis 60 Gew.% so vermischt, daß die Menge des Polymeren 20 g je 100 g Ferrit beträgt. Die so hergestellte Suspension wird eine Stunde lang mit einem Ultra Turrax Dispergieraggregat unter der Wirkung hoher Scherkräfte eine halbe bis zwei Stunden dispergiert. Hierbei kann die Temperatur bis zu 70 °C ansteigen. Die Reihenfolge der Zugabe der Komponenten ist beliebig und wirkt sich nicht auf die Eigenschaften des resultierenden Tintenkonzentrats aus. Anschließend wird 5 Minuten bis zwei Stunden bei 200 bis 2000 g zentrifugiert und der kleine Anteil sedimentierter Teilchen abgetrennt. Das resultierende Produkt entspricht in Aufbau und Eigenschaften dem erfindungsgemäßen magnetischen Tintenkonzentrat.

In seinem Eigenschaftsprofil ist das erfindungsgemäße Tintenkonzentrat genauso sedimentationsstabil wie das in der mehrfach genannten DE-OS 41 15 608, das heißt, nach einer einwöchigen Lagerung erhöht sich die Konzentration des Farbstoffs am Boden einer beispielsweise 10 cm hohen Flüssigkeitssäule um weniger als 3 % gegenüber der mittleren Konzentration.

Das Sedimentationsverhalten wurde mit einem ortsauflösenden Suszeptibilitätsmeßgerät gemessen und die monotone Verteilung der magnetischen Substanz bestimmt.

Außerdem zeichnet sich das Tintenkonzentrat dadurch aus, daß es bei der Handhabung nicht schäumt und zu keinem Zeitpunkt Klumpen bildet und außerdem geruchsarm ist. Es eignet sich daher in hervorragender Weise als magnetische Tinte für Schreibgeräte wie etwa Tintenstrahldrucker, wobei das entstandene Schriftbild scharf und nicht verfließend und wischfest ist. Auch läßt sich das erfindungsgemäße magnetische Tintenkonzentrat zur Informationsspeicherung mittels magnetischer Strichkode nutzen, da es die hohe Koerzitivkraft des Pigments und seine Remanenz hierfür besonders geeignet macht.

Weitere Anwendungsmöglichkeiten, welche für das erfindungsgemäße Tintenkonzentrat in Betracht kommen, sind in der Medizin als MRI-Kontrastmittel, wie in der PCT-Anmeldung O.Z. 0050/43254 beschrieben sowie auch technische Anwendungen beispielsweise Polieren mit einer Mischung aus Tintenkonzentrat und Schleifmittel im Magnetfeld.

### Beispiel 1

### Ferritherstellung

Eine Lösung aus 216,7 g FeCl₃ x 6H₂O, 49,9 g FeCl₂ x 4H₂O, 49,9 g CoCl₂ x 4H₂O, 6 ml 10%iger HCl und 440 ml Wasser wurde bei 25 °C innerhalb von 10 Minuten zu einer Lösung von 113 g NaOH und 440 ml Wasser getropft. Der pH nach Ende der Fällung betrug 11,5. Die Suspension wurde auf 50 °C erwärmt und 1 Stunde bei dieser Temperatur belassen. Nach Abkühlen wurde ein pH von 9 eingestellt. Es wurde abgesaugt und der Ferritfilterkuchen mit 1,5 l Wasser gewaschen. Ein Teil des Ferrits wurde zur Charakterisierung getrocknet; es wurden folgende Eigenschaften gemessen: BET = 103 m²/g, H_{c} = 21 kA/m, Mₘ/ = 72 nTm³/g, Mᵣ/ = 12 nTm³/g.

### Herstellung des Tintenkonzentrats

163 g des Filterkuchens (mit 40 g Kobaltferrit) wurden mit einer Lösung von 8 g des Natriumsalzes der Polyacrylsäure (mit einem Molekulargewicht von 4000) und 10 g Wasser vermischt. Es wurde ein pH zwischen 9 und 10 eingestellt und 1 Stunde lang mit einem Ultra Turrax Dispergieraggregat (Typ 18G) bei maximaler Drehzahl dispergiert. Anschließend wurden leichtsedimentierende Bestandteile durch 5minütige Zentrifugation bei 2000 g Beschleunigung abgetrennt. Die Ausbeute nach der Zentrifugation betrug 78 %.

### Tintenformulierung

Für den Druck mit einem Tintenstrahldrucker (HP DeskJet 500) wurde folgende Tintenformulierung gewählt: 41 g des Tintenkonzentrats wurden mit 2 g Diethylenglykol, 3 g Tripropylenglykolmonomethylether und 54 g Wasser gemischt.

### Magnetik des Drucks

Es wurde eine kleine Fläche Papier mit Tinte bedruckt und die magnetischen Eigenschaften bestimmt. Der Druck ist zur magnetischen Informationsspeicherung geeignet. Die H_{c} betrug 16 kA/m.

### Beispiel 2

### Ferritherstellung

Das Bariumferrit wurde nach dem bekannten Glasverfahren hergestellt. Es wurde als feuchter Filterkuchen verarbeitet. Ein Teil des Ferrits wurde zur Charakterisierung getrocknet; es wurden folgende Eigenschaften gemessen: BET = 104 m²/g, H_{c} = 106 kA/m, Mₘ/ = 35 nTm³/g, Mᵣ/ = 15 nTm³/g.

### Herstellung des Tintenkonzentrats

40 g des Filterkuchens (mit 9 g Bariumferrit) wurden mit einer Lösung von 1,8 g des Natriumsalzes der Polyacrylsäure (mit einem Molekulargewicht von 4000) und 2,2 g Wasser vermischt. Es wurde pH 10 eingestellt und 1 Stunde lang mit einem Ultra Turrax Dispergieraggregat (Typ 18G) bei maximaler Drehzahl dispergiert.

### Tintenformulierung

Für den Druck mit einem Tintenstrahldrucker (HP DeskJet 500) wurde folgende Tintenformulierung gewählt: 20 g des Tintenkonzentrats wurden mit 0,82 g Diethylenglykol, 1,23 g Tripropylenglykolmonomethylether und 19 g Wasser gemischt.

### Magnetik des Drucks

Es wurde eine kleine Fläche Papier mit Tinte bedruckt und die magnetischen Eigenschaften bestimmt. Der Druck ist zur magnetischen Informationsspeicherung geeignet.

### Beispiel 3

### Ferritherstellung

Eine Lösung aus 437,7 g FeCl₃ · 6 H₂O, 99,9 g FeCl₂ · 4 H₂O, 101,2 g CoCl₂ · 4 H₂O, 12 ml 10%iger HCl und 880 ml Wasser wurde bei 25 °C innerhalb von 20 Minuten zu einer Lösung von 272,5 g NaOH und 880 ml Wasser getropft. Es wurde 15 Minuten lang gerührt. Der pH wurde von 12,1 durch HCl Zugabe auf 10 eingestellt. Die Suspension wurde auf 70 °C erwärmt und 2 Stunden bei dieser Temperatur belassen. Es wurde abgesaugt und der Ferritfilterkuchen mit 3 l Wasser gewaschen. Ein Teil des Ferrits wurde zur Charakterisierung getrocknet; es wurden folgende Eigenschaften gemessen: BET = 91 m²/g, H_{c} = 35 kA/m, M_{m/} = 75 nTm³/g, M_{r/} = 20 nTm³/g.

### Herstellung des Tintenkonzentrats

552 g des Filterkuchens (mit 179 g Ferrit) wurden mit einer Lösung von 35,8 g des Natriumsalzes der Polyacrylsäure (mit einem Molekulargewicht von 4000) und 42 g Wasser sowie mit 448 g Ethylenglykol vermischt. Es wurde ein pH zwischen 9 und 10 eingestellt und 1 Stunde lang mit Eiskühlung mit einem Ultra Turrax Dispergieraggregat (Typ 18G) bei maximaler Drehzahl dispergiert. Anschließend wurden leichtsedimentierende Bestandteile durch 5minütige Zentrifugation bei 2000 g Beschleunigung abgetrennt. Die Ausbeute nach der Zentrifugation betrug 71 %. Durch Trocknung am Rotationsverdampfer bei 80 °C/Vakuum wurde das Wasser entfernt. Es entstand eine sedimentationsstabile Suspension: keine Sedimentbildung nach zweiwöchiger Lagerung. Die Suspension ist für den Einsatz in Schreibgeräten, die auf der Wirkung kapillarer Kräfte beruhen, geeignet.

## Patentansprüche

1. Magnetisches Tintenkonzentrat, im wesentlichen bestehend aus einer Dispersion magnetischer Feststoffteilchen in Wasser oder Alkohol in Gegenwart dispergierender Substanzen, wobei die dispergierende Substanz aus mindestens einem Polyelektrolyten mit einem Molekulargewicht zwischen 1000 und 25000, ausgewählt aus der Gruppe aus Polyacrylat, Acrylsäure-/Acrylamid-Copolymeren und Polyvinylphosphonsäure sowie der Alkalisalze dieser Verbindungen besteht, dadurch gekennzeichnet, daß die magnetischen Feststoffteilchen der Formel MₐMn_{b}Zn_{c}Fe_{d}Oₑ gehorchen, wobei
M = Co und/oder Ni und
a, b, c = 0,000 - 0,99 a+b+c = 0,01 - 0,99
d = 2,00 - 2,99 a+b+c+d = 2,00 - 3,00
e = 3,01 - 4,
mit der Maßgabe, daß die magnetischen Feststoffteilchen eine Koerzitivkraft im Bereich von 10 bis 500 kA/m, eine Remanenz von größer als 10 nTm³/g und eine spezifische Oberfläche von 70 bis 200 m²/g besitzen.

2. Magnetisches Tintenlkonzentrat, im wesentlichen bestehend aus einer Dispersion magnetischer Feststoffteilchen in Wasser oder Alkohol in Gegenwart dispergierender Substanzen, wobei die dispergierende Substanz aus mindestens einem Polyelektrolyten mit einem Molekulargewicht zwischen 1000 und 25000, ausgewählt aus der Gruppe aus Polyacrylat, Acrylsäure-/Acrylamid-Copolymeren und Polyvinylphosphonsäure sowie der Alkalisalze dieser Verbindungen besteht, dadurch gekennzeichnet, daß die magnetischen Feststoffteilchen aus der Gruppe der hexagonalen Barium-, Strontium- oder Calziumhaltigen Ferrite sind, mit der Maßgabe, daß die magnetischen Feststoffteilchen eine Koerzitivkraft im Bereich von 10 bis 500 kA/m, eine Remanenz von größer als 10 nTm³/g und eine spezifische Oberfläche von 70 bis 200 m²/g besitzen.

3. Magnetisches Tintenkonzentrat nach Anspruch 1 oder 2, dadurch gekennzeichnet, daß den magnetischen Feststoffteilchen bis zu 50 Gew.% superparamagnetische und/oder unmagnetische feinteilige Feststoffpigmente zugemischt sind.

4. Magnetisches Tintenkonzentrat nach Anspruch 1 bis 3, dadurch gekennzeichnet, daß als Polyelektrolyte auch Polykondensate aus organischen Hydroxy-Carbonsäuren, HydroxyCarbonsäuren und Alkoholen oder Acetalen eingesetzt sind, wobei das Molekulargewicht der Monomere zwischen 100 und 400 liegt, wobei der K-Wert einer 1%igen Lösung der Polymeren in Wasser zwischen 10 bis 50 beträgt und wobei im Mittel wenigstens 5 Ladungsträger in der Polymerkette vorhanden sind.

5. Magnetisches Tintenkonzentrat nach Anspruch 1 oder 2, dadurch gekennzeichnet, daß der Alkohol eine Dielektrizitätskonstante von mindestens 20 besitzt.

6. Verfahren zur Herstellung der magnetischen Tintenkonzentrate gemäß Anspruch 1 bis 5, dadurch gekennzeichnet, daß die hartmagnetischen Feststoffteilchen durch Zugabe einer 20 bis 60 Gew.%igen Lösung des Polyelektrolyten in Wasser oder Alkohol zu dem feuchten Filterkuchen dieser Feststoffteilchen suspendiert, diese Suspension anschließend unter Einwirkung hoher Scherkräfte dispergiert und durch Zentrifugieren von dem Anteil sedimentierter Teilchen getrennt wird.

7. Verwendung der magnetischen Tintenkonzentrate gemäß den Ansprüchen 1 bis 5, zur Fixierung von Informationen mittels Tintenstrahldrucker und Strichkodes.

8. Verwendung der magnetischen Tintenkonzentrate gemäß den Ansprüchen 1 bis 5 als Kontrastmittel in der Medizin (MRI).

9. Verwendung der magnetischen Tintenkonzentrate gemäß den Ansprüchen 1 bis 5 als Poliermittel einer Mischung aus magnetischem Tintenkonzentrat und Schleifmitteln unter Einwirkung eines Magnetfeldes.
